# EUROPEAN PATENT APPLICATION

(11) **EP 4 292 665 A1**
(43) Date of publication of application: **20.12.2023**
(21) Application number: 22198954.4
(22) Date of filing: 30.09.2022
(51) Int. Cl.: A61Q 11/00, A61K 8/81, A61K 8/64, A61K 8/46, A61K 8/44, A61K 8/24, A61K 8/19, A61K 8/04, A61K 6/20

(54) **AN INSTANTLY FORMED CATIONIC ACP GEL FOR TREATMENT OF TOOTH HYPERSENSITIVITY**

(30) Priority: 16.06.2022 US 202263352752 P
(71) Applicant: Koninklijke Philips N.V., 5656 AG Eindhoven (NL)
(72) Inventor: MAO, Jingliang, Eindhoven (NL); GODDARD, Gregory Russ, Eindhoven (NL); CHEN, Wan-Tzu, 5656AG Eindhoven (NL); MIRZA, Farah, 5656AG Eindhoven (NL)
(74) Representative: Philips Intellectual Property & Standards

(57) **Abstract**

An composition for treating dentin hypersensitivity including an instantly formed cationic amorphous calcium phosphate (ACP) gel, methods for using the composition, and systems for preparing the gel composition.

## Description

### FIELD OF THE INVENTION

The present disclosure relates generally to compositions for treating dentin hypersensitivity, related oral health care methods, and systems for preparing such compositions.

### BACKGROUND OF THE INVENTION

Tooth and gum sensitivity are areas of particular concern in oral health care, usually the most prominent concern after tooth cavities. As more and more people use teeth whitening products, this concern is growing.

Dentin hypersensitivity is characterized as a sharp short pain arising from exposed dentin, typically in response to an external stimulus such as clinical, thermal, tactile, evaporative or osmotic stimuli. It is often experienced when dentin is exposed to the oral environment because of gingival recession or a loss of enamel.

The most widely accepted explanation of dentin hypersensitivity is provided by the hydrodynamic theory, which suggests that nerve stimulation due to movement of dentinal fluid is the cause of the hypersensitivity. When dentin tubules are exposed to the oral environment, fluid movement through dentin tubules, can be sensed by internal tooth nerves. The activation of tooth nerve fibers can cause the feeling of sharp pain. The relationship between dentin hypersensitivity and the patency of dentin tubules has been established in vivo and it has been found that occlusion of the tubules decreases that sensitivity.

In general, the technology and materials developed to treat dentin hypersensitivity can be classified into two treatment categories: Treatment I and Treatment II described below.

Treatment I works by desensitizing the tooth nerves via potassium salts like potassium nitrate, potassium citrate, potassium chloride, which depolarize the excited nerve fibers, thus numbing the pain. This treatment typically provides short-term relief of dentin hypersensitivity.

Treatment II is based on occlusion of the dentin tubules by plugging dentinal tubules or coating the sensitive dentin area with a thin protective layer of material. Occlusion of open tubules blocks the hydrodynamic mechanism. Treatment II may provide a relatively long-term solution.

Most available dentin hypersensitivity treatment products and methods work by blocking the dentinal tubules and prevent dentinal fluid flow. Examples of such materials and methods include the application of specific dentifrices, dentin adhesives, some antibacterial agents, aldehydes, resin suspensions, fluoride rinses, fluoride varnishes, amorphous calcium phosphate (ACP), strontium fluorides, oxalates, Nd-YAG laser application, bioactive glass, casein phosphopeptides, and Portland cement.

In general, the existing products and methods to treat dentin hypersensitivity have one or more of the problems or disadvantages described below.

Existing products typically provide insufficient blockage of open tubules in a depth-wise direction of the tubules as well as in terms of area coverage of the openings of the dentin surface. Existing products generally provide slow diffusion and/or dispersion of the active occlusion ingredients into the open dentin tubules. For example, in products that use an arginine bicarbonate calcium carbonate complex to plug dentinal tubules, the arginine binds onto tooth surfaces and recruits calcium and phosphate ions from saliva. Since calcium carbonate has a low solubility in water to produce calcium ions and the tooth dentin microtubules are around 5 µm in diameter, it takes time to build up effective plugging with compounds formed by calcium and phosphate ions inside the dentin tubules.

Existing products offer weak or low affinity to dentin and provide unstable remineralization and lower efficiency of occlusion of dentin tubules. For example, calcium and phosphate ions may be released from a pre-formed amorphous calcium phosphate (ACP) gel in the presence of saliva to accelerate the uptake of these bioavailable ions, but the formed compounds lack stability and have low affinity to dentin inside dentin tubules.

Existing products provide hypersensitivity relief only for a limited time. For example, it has been reported that a composition of arginine, bicarbonate and calcium carbonate is burnished onto sensitive teeth following scaling and root planing procedures in a dental clinic, and the reported sensitivity relief lasts for at least 28 days by a single treatment (D. Cummins, Dentin hypersensitivity: from diagnosis to a breakthrough therapy for everyday sensitivity relief, J Clin Dent. 2009;20(1): 1-9).

Existing products and methods are inconvenient, costly and/or uncomfortable treatments. Many existing dentin hypersensitivity treatments require dental clinic visits.

In view of the foregoing, there is a persisting need to improve products and techniques to treat dentin hypersensitivity.

### SUMMARY OF THE INVENTION

According to the implementations and embodiments described herein addressing such a need, compositions and methods for treating dentin hypersensitivity are provided, as well as systems for preparing such compositions.

The disclosure is directed to a composition for treating dentin hypersensitivity, including a first component and a second component. The first component includes a pH sensitive rheology modifying polymer, a calcium ion source, and an acidifying agent. The first component has a pH in the range of about 2.0 to about 6.5. The second component includes a phosphate ion source, a positively charged basic amino acid and/or a cationic peptide, and an alkalizing agent. The second component has a pH in the range of about 9.5 to about 14.0. The first component and the second component are combined into a mixture, for example via a mixing tip or chamber, creating a cationic amorphous calcium phosphate (ACP) gel composition instantly when the pH of the mixture reaches a pH range of about 7.0 to about 9.5.

An object of the inventive subject matter is to provide an instantly formed ACP relief gel, including cationic amino acids, protein and/or peptides, that provides stronger dentin affinity than existing products and forms better occlusion aggregates to treat dentin hypersensitivity more effectively.

In some embodiments, the pH sensitive rheology modifying polymer can be selected from a hydrophobically modified alkali-soluble emulsion (HASE) polymer of polyacrylic acid, an alkali-soluble emulsion (ASE) polymer of polyacrylic acid, and a polymer with one or more acidic groups selected from the group consisting of carboxylic acid, sulfonic acid, and phosphoric acid, and capable of thickening a composition including water upon an increase of the pH of the composition from an acidic to an alkaline value within a pH range of about 7.0 to about 12.0.

In some embodiments, the calcium ion source is a calcium salt of nitric acid, a calcium salt of aspartic acid, a calcium salt of glutamic acid, or a water-soluble calcium salt of other suitable acid. In some embodiments, the acidifying agent is phosphoric acid, or other acid that is compatible with ingredients of the first component. In other embodiments, the phosphate ion source is disodium phosphate, monosodium phosphate, or other suitable alkaline water-soluble phosphate salt. In some embodiments, the positively charged basic amino acid is arginine, lysine, histidine, or combinations thereof. In some embodiments, the cationic peptide is a water-soluble peptide that is positively charged at neutral pH and non-toxic for a user. In some embodiments, the alkalizing agent is sodium hydroxide, potassium hydroxide, or other suitable alkaline compound. In further embodiments, viscosity of the instantly formed cationic ACP gel is larger than about 5,000 cP.

The subject matter disclosed herein is further directed to an oral health care method for treating dentin hypersensitivity, including providing a first component and a second component. The first component includes a pH sensitive rheology modifying polymer, a calcium ion source, and an acidifying agent. The first component has a pH in the range of about 2.0 to about 6.5. The second component includes a phosphate ion source, a positively charged basic amino acid and/or a cationic peptide, and an alkalizing agent. The second component has a pH in the range of about 9.5 to about 14.0. The method further includes separately housing the first component and the second component, dispensing the first component and the second component in a mixing tip or chamber thereby combining the first component and the second component into a mixture forming an ACP gel composition instantly when the pH of the mixture reaches a pH range of about 7.0 to about 9.5, and applying the instantly formed cationic ACP gel composition onto the dentin surface of the user's teeth.

In some embodiments, the pH of the first component is in the range of about 2.5 to about 4.5. In some embodiments, the pH of the second component is in the range of about 10.5 to about 12.5. In further embodiments, the pH of the instantly formed cationic ACP gel composition ranges from about 7.0 to about 9.5.

The subject matter described herein further includes a system for preparing a composition for treatment of dentin hypersensitivity, including a dosing device and a dispensing device. The dosing device includes a first compartment containing a first component including a pH sensitive rheology modifying polymer, a calcium ion source, and an acidifying agent, and wherein the first component has a pH in the range of about 2.0 to about 6.5, and a second compartment containing a second component of a phosphate ion source, a positively charged basic amino acid and/or a cationic peptide, and an alkalizing agent, and wherein the second component has a pH in the range of about 9.5 to about 14.0. The dispensing device is configured to receive the first component and the second component into a mixing tip or chamber and to combine the first component and the second component into a mixture creating an ACP gel composition instantly when the pH of the mixture reaches a pH range of about 7.0 to about 9.5, and wherein the dispensing device has an opening to apply the cationic ACP gel composition onto a dentin surface of a user's teeth.

In some embodiments, the dispensing device is adapted to dispense the cationic ACP gel composition as a viscous gel with a viscosity larger than about 5,000 cP. In other embodiments, the dosing device includes multiple compartments, each compartment comprising one or more compatible ingredients of the first component and/or one or more compatible ingredients the second component, and wherein the content of the multiple compartments is dispensed into the mixing tip or chamber to form the cationic ACP gel composition. In some embodiments, ingredients for each compartment are supplied in cartridge form. In further embodiments, the dispensing device can include a dual-barrel syringe, dual-chamber bottle, or a form of a dispensing device that can dispense two separate components into a common mixing tip to form the cationic amorphous calcium phosphate gel composition.

These and other aspects of the disclosure will be apparent from and elucidated with reference to the embodiment(s) described hereinafter.

### BRIEF DESCRIPTION OF THE DRAWINGS

FIG. 1 is perspective view of an exemplary system using a dual-barrel syringe and mixing tip to form an instant cationic ACP gel composition.
FIG. 2 is a simplified front view of an exemplary system using a dual-chamber bottle and mixing tip to form an instant cationic ACP gel composition.
FIG. 3 is a schematic diagram of an exemplary system wherein the dosing device includes three compartments.
FIG. 4 is a high-level flow chart of an exemplary method for treating dentin hypersensitivity.

### DETAILED DESCRIPTION OF EMBODIMENTS

A composition for treating dentin hypersensitivity is disclosed, as well as methods for making such compositions, and systems for preparing compositions. The disclosed compositions include an instantly formed cationic amorphous calcium phosphate (ACP) gel that has several advantages over current market available ACP gels that are not cationic and/or are not instantly formed.

It is known that dentin or tooth surface is negatively charged in nature. Chemically, dentin is composed, by weight, of approximately 20% of organic material, approximately 75% of inorganic material, and approximately 5% water. The organic material is primarily collagen, which functions as a binder in the dentin structure. The inorganic material includes calcium phosphate.

In healthy teeth, saliva is naturally effective in reducing dentin hypersensitivity by supplying and carrying calcium and phosphate ions into the open dentin tubules to gradually occlude them and to form a surface protective layer consisting of precipitate of salivary glycoproteins with calcium phosphate. For people having dentin hypersensitivity, the affected tooth dentin exposes numerous microtubules of around 5 µm in diameter. The microtubules allow small molecular size fluid or particles to migrate inside the dentin structure. It is highly beneficial to have a composition of a dentin occluding material close or similar to dentin's natural composition, i.e., being made of calcium phosphates; amino acids, peptides, or proteins; and water. Thus, a cationic charged, instantly formed ACP gel is desired in terms of freshness, binding affinity, and occlusion efficiency of dentin tubules.

As disclosed herein, a composition for treating dentin hypersensitivity includes a first component including a pH sensitive/induced rheology modifying polymer, a calcium ion source, and an acidifying agent, and a second component including a phosphate ion source, a positively charged basic amino acid and/or a cationic peptide, and an alkalizing agent. The first component has a pH in the range of about 2.0 to about 6.5. The second component has a pH in the range of about 9.5 to about 14.0. The first component and the second component are combined into a mixture via a mixing tip or chamber, whereby the cationic ACP gel composition is created instantly when the pH of the mixture reaches a pH range of about 7.0 to about 9.5. A new instant gelling approach is disclosed that uses pH manipulation to produce a fresh, cationic charged, ACP gel in an instantly gelling manner. By using the methods, systems, and compositions disclosed herein, not only are problems of existing technologies addressed, but also a longer dentin hypersensitivity relief effect can be achieved in a natural, convenient, and cost-effective way.

As used herein, the term "instant" and "instantly" refer to the short time from the time of mixing the components to the time of formation of the finished gel, which is generally less than about 5 seconds.

As used herein, the term "gel" refers to a homogenous solid aqueous gel-like material, such as a hydrogel having water-swellable polymeric matrices that can absorb a substantial amount of water.

The term "acidifying agent" refers to one or more compounds that when dissolved or dispersed in neutral water having a pH of 7 causes the water to have a pH lower than 7. It may refer to Bronsted or Arrhenius acid.

The term "alkalizing agent" refers to one or more compounds that when dissolved or dispersed in neutral water having a pH of 7 causes the water to have a pH higher than 7.

As used herein, the term "pH manipulation" refers to a formulation strategy accomplished by a pH change of the mixture when a pH sensitive thickener is used in a multi-component composition. The pH manipulation is conducted by raising the pH of the final mixture to an alkaline pH, in particular a pH of about 7.0 to about 12.0, when a predetermined amount of one acidic component with pH sensitive thickener is mixed with an amount of another alkaline component via a mixing tip or in a mixing chamber. During the mixing, the acidic pH sensitive thickener molecules will be deprotonated, and an instant thickening power is created by the electrostatic repulsion among charges present on the deprotonated polymer chain. The use of HASE polymers and/or ASE polymers that are not crosslinked in the component form allows for rapid gel formation in the mixture, which can be instantly.

The term "thickening" refers to an increase in the viscosity of the composition. A pH sensitive thickener is capable of increasing the viscosity of a composition upon subjecting it to an increasing pH such as in particular the composition pH in the range of 7.0 to 12.0. The thickener is compatible with one or more of the basic amino acids/peptides, phosphate ions, calcium ions, and/or acidic amino acids.

In mechanism, when the pH of an acidic component containing pH sensitive rheology modifying polymers, such as hydrophobically modified alkali-soluble emulsion (HASE) polymers or other alkali-swellable acrylic (ASE) polymers, goes above pH 7.0, and below pH 12.0, after being neutralized with an alkaline component of phosphate ions and basic amino acids or cationic peptides, it can instantly thicken and turn the final mixture into a cationic gel with large quantities of calcium and phosphate ions and some instantly formed ACP nano scale particles. For applications on humans, a pH of about 7.0 to about 9.5 of the resulting cationic ACP gel is desired.

As a pH sensitive rheology modifier, HASE polymers swell first due to charge-charge repulsion when its acid groups are neutralized/deprotonated with inorganic bases or organic amines, and therefore thickens instantly in response to an increased pH. With these polymers swelling, the pendant hydrophobic groups are then freed to build associations with one another and/or with other available hydrophobic groups in the system. This phenomenon creates a network structure that results in a significant viscosity increase, which further boosts the gelling effect, and stabilizes and disperses any particulates, which may be present in the composition.

In an example embodiment, the pH-sensitive thickener can include one or more polymers each having acidic groups. Such acidic groups will be in protonated form at the first acidic pH and in deprotonated form at the final mixture pH in the range of about 7.0 to about 12.0. The deprotonation provides charged sites which are responsible for the polar interactions for thickening.

The inventive subject matter takes advantage of pH sensitive thickening polymers and the interaction principle of charged particles in physics: opposites attract, and likes repel. It also takes advantage of the chemical properties of calcium salts of aspartic acid or glutamic acid, or cationic charges of lysine, arginine, and histidine which are readily soluble in water at neutral pH.

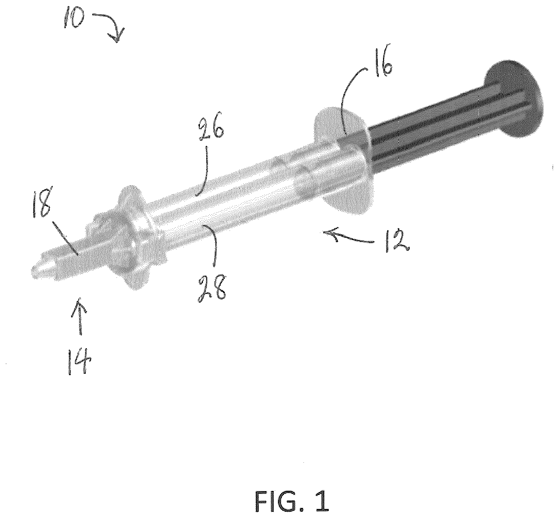

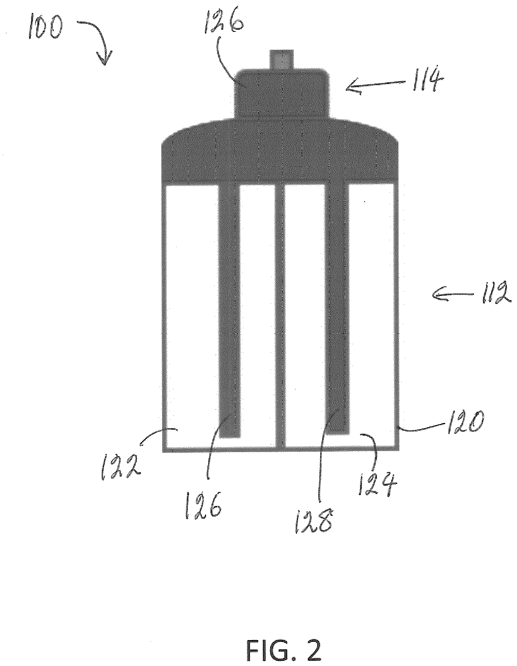

The inventive subject matter is directed to a system for preparing a composition for treatment of dentin hypersensitivity, including a dosing device and a dispensing device. The dosing device includes at least two compartments. Examples of embodiments using a dosing device including two compartments are shown in FIG. 1 and FIG. 2. A first compartment includes a first component of pH sensitive rheology modifying polymer, a calcium ion source, and an acidifying agent. A second compartment includes a second component of a phosphate ion source, a positively charged basic amino acid and/or a cationic peptide, and an alkalizing agent. The dispensing device is configured to receive the first component and the second component into a mixing tip or chamber in which the respective components may be combined or mixed to form a mixture of the cationic ACP gel composition instantly when the pH of the mixture reaches a pH range of about 7.0 to about 9.5. The resulting mixture is freshly made on-site and can be retrieved from the chamber via an opening and applied onto a dentin surface of a user's teeth.

In an example embodiment, a cationic ACP gel composition can be formed by the combination of predetermined quantities of two components, Part 1 and Part 2, as described below.

**TABLE 1**

| Composition of two-part system for instantly formed, cationic, ACP gel for treatment of a hypersensitive tooth | |
|---|---|
| Part 1 | Solution of pH sensitive polymer(s) like HASE polymer, calcium salts of nitric acid, aspartic acid (Asp), glutamic acid (Glu) or other suitable acid, acidifying agents, plus other functional or inert ingredient(s), pH 2.0-6.5, preferably 2.5-4.5. |
| Part 2 | Solution of disodium phosphate, basic amino acids like arginine (Arg), lysine (Lys), and/or histidine (His), and/or cationic peptide, alkalizing agents, plus other functional or inert ingredient(s), pH 9.5 -14.0, preferably 10.5 - 12.5. |

The two-part system in solution form has the following benefits. A calcium salt, like a calcium nitrate salt, is soluble in acidic environment but not stable in alkaline conditions. A phosphate salt, like disodium phosphate, is very soluble in strong alkaline condition but less soluble in a neutral pH. When the two parts are mixed under the right ratios and conditions, ACP formation starts around neutral pH and accelerates with increased pH, since ACP has very poor solubility in neutral or slight alkaline pH condition. Furthermore, basic amino acids like arginine, lysine, and histidine are water soluble, and positively charged in a neutral or near neutral pH environment.

Part 1 and Part 2 can be in liquid form, either as a solution or as an emulsion. The mixing can be accomplished by using a mixing tip, as further discussed below with reference to FIG. 1, or as by using a mixing chamber, as further discussed below with reference to FIG. 2. By using both Part 1 and Part 2 in liquid form, the mixing of the components is easier, results in the component's ingredients to be more homogeneously mixed and provides faster viscosity increase. In some embodiments, the viscosity of the resulting cationic ACP gel composition is larger than 5,000 cP, preferably larger than 10,000 cP.

FIG. 1 illustrates an embodiment wherein a system 10 includes a dosing device 12 and a dispensing device 14. Dosing device 12 includes dual reservoirs, each one of the reservoirs holding a fluid component at the desired mixing ratios. Dosing device 12 consists of a dual-barrel syringe 16 wherein one barrel 26 contains Part 1 and the other barrel 28 contains Part 2. Dispensing device 14 has a mixing tip 18 wherein Part 1 and Part 2 are mixed to form an instant cationic ACP gel composition.

Such a system allows the alkaline and the acidic components to be stored separately before use. It allows for ingredient stability and avoids premature reaction or binding among ingredients of the two separate components before they reach the targeted area. When needed, the alkaline and the acidic components can be mixed and gelled from a single unit at a joint mixing point or chamber before starting of applications.

FIG. 2 illustrates another embodiment wherein a system 100 includes a dosing device 112 and a dispensing device 114. Dosing device 112 includes a dual-chamber bottle 120, each chamber 122, 124 holding a fluid component at the desired mixing ratios. The components are separated by a wall 126 and each chamber 122, 124 includes a pipe 126, 128 to dispense the content of each chamber 122, 124 into dispensing device 114 which includes a mixing tip 126 wherein the instant cationic ACP gel composition is formed. The viscous gel can be applied onto the user's teeth or onto an exposed dentin surface.

Optionally, the dosing system can include a pump or vacuum means to simultaneously withdraw fluid from each compartment, for example via separate draw pipes, and discharge the fluid into a common mixing chamber and after that the instantly formed gel content to the application areas.

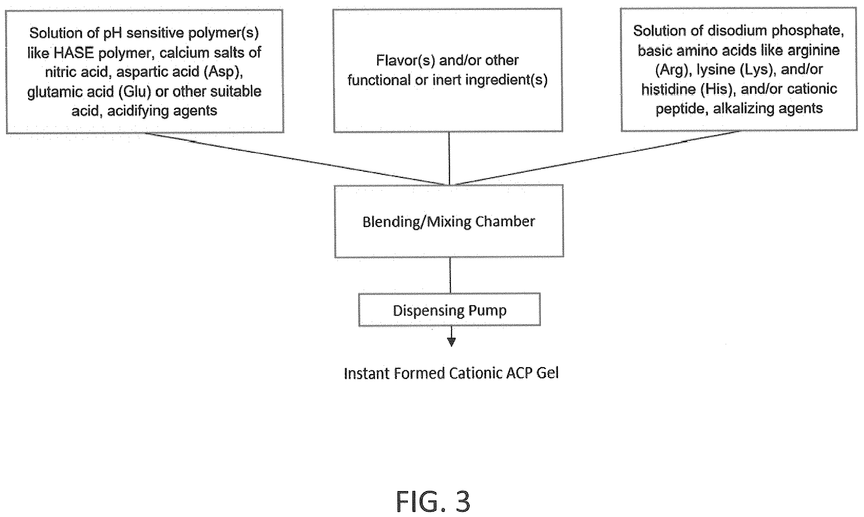

In other embodiments, a cationic ACP gel composition can be formed by combining predetermined quantities of multiple components such as three parts, four parts, or multiple parts. FIG. 3 shows a diagram of a system wherein the dosing device includes three compartments. A first compartment can include for example Part 1 as described above. A second compartment can include Part 2 as described above. A third compartment can include one or more flavors and/or other functional or inert ingredients. The content of the three compartments is combined in a blending or mixing chamber. The resulting cationic ACP gel composition can be removed from the chamber via a dispensing pump and applied to a dental area that is to be treated.

In some embodiments, the dispensing system can be designed to work similar to a soda fountain with multiple components. Such a system can accurately control dosage of each component and/or ingredient. In some embodiments, each component/ingredient can be supplied in cartridge form or as replaceable containers to make it easier for dosing control, replacement, change, or product choice during application.

The composition can be made on-site, i.e., at the location of use shortly prior to administration, to preserve freshness and effectiveness. In some embodiments, the instantly formed cationic ACP gel can be applied directly to exposed dentin for dentin hypersensitivity relief, for example by massaging the composition for 1 to 2 minutes directly onto the exposed dentin to help diffusion and/or dispersion into dentin microtubules. In some embodiments, this can be repeated once or twice a day to obtain the desired effect.

The inventive subject matter will be more readily understood by referring to the following detailed descriptions of ingredients and formulations which are given to illustrate the disclosure rather than to limit its scope.

As described above, a cationic ACP gel composition can be made of two components, Part 1 and Part 2. The combination of these components provides more effective hypersensitivity relief than just ACP alone.

For the pH sensitive polymers used in Part 1, there are many non-hazardous, pH sensitive rheology modifying polymers suitable. These polymers are provided in a form that is not crosslinked and only thicken compositions substantially at alkaline pH. Such polymers include HASE polymers, acrylic ASE co-polymers, such as those commercially available family of polymers sold under the tradename Carbopol^{®}, acrylate copolymers, and polymers with one or more acidic groups selected from the group consisting of carboxylic acid, sulfonic acid, and phosphoric acid, and capable of thickening a composition including water upon an increase of the pH of the composition from an acidic to an alkaline value within a pH range of about 7.0 to about 12.0.

One example of suitable acrylic ASE co-polymers from the Carbopol^{®} series is Carbopol^{®} 940 NF polymer. The pH of a 1% Carbopol^{®} 940 NF solution is about 2.5 - 3.0 and the viscosity is well below 1500 cP under that pH. However, it produces 40,000 - 60,000 cP of viscosity at 0.5% dispersions under pH 7.5- 10.0. A 1% neutralized mucilage of Carbopol^{®} 940 NF polymer forms a stiff gel. A preferred dosage of CARBOMER^{®} 940 NF and other suitable Carbopol^{®} polymers is between 0.5- 3.0 % solid polymer levels.

Another suitable example is HASE polymers synthesized from an acid/acrylate copolymer backbone and including an ethoxylated hydrophobe. One example of HASE polymers is ACULYN^{™} 28. It can form a highly viscous gel, having a viscosity of more than 100,000 Cp, at pH 7.0 - pH 12 with 5% (wt.) ACULYN^{™} 28 dosage whereas very little viscosity is exhibited, i.e., less than 30 Cp for 100% ACULYN^{™} 28) at pH 3.0. In some embodiments, the preferred dosage level of ACULYN^{™} 28 and other HASE polymers can be between 1%- 10% level in the final mixed gel, from a cost-effect perspective.

The calcium ion source in Part 1 may include calcium salts of nitric acid, aspartic acid, glutamic acid or other suitable acid such as calcium nitrate, aspartic acid calcium, or calcium glutamate could be used, as long as it remains soluble at pH 2.0-6.0 in a water solution. The preferred dosage range of calcium salts is 0.5%- 1.5% w/w.

The phosphate ion source in Part 2 can include disodium phosphate, monosodium phosphate or other alkaline water soluble, non-toxic phosphate salts can be used in the formulation of Part 2. The preferred dosage range is 0.5%-1.5% w/w.

Positively charged basic amino acids and/or a cationic peptides in Part 2 include the three basic amino acids, lysine, arginine, and histidine, which have basic side chains at neutral pH. Table 2 shows their pKa1(α-carboxyl group), pKa2 (a-ammonium ion), pKa3 (side chain R group) and isoelectronic point PI. The basic amino acids are positively charged at neutral pH condition. Especially arginine and lysine have 100% positively charged basic side chains between pH 6.5-8.0 which makes these amino acids particularly suitable. Depending on which basic amino acid is used in the composition, it is practical to have strong positively charged basic amino acids around neutral pH. Among the two optical isomers, D(-) and L(+), available of lysine, arginine, or histidine, the L form of lysine, arginine, or histidine is preferred. The preferred dosage range of each basic amino acid is 6-12% w/w. In formulation, the pH of Part 2 solution should be kept away from the PI value when the relevant basic amino acid is used in Part 2. The pKa1, pKa2, pKa3 and PI of lysine, arginine, and histidine, respectively are listed in Table 2 below.

**TABLE 2**

| Basic Amino Acid | pKa1 (α-carboxyl group) | pKa2 (a-ammonium ion) | pKa3 (side chain R group) | pI (isoelectronic point) |
|---|---|---|---|---|
| Lysine | 2.18 | 8.95 | 10.53 | 9.74 |
| Arginine | 2.17 | 9.04 | 12.48 | 10.76 |
| Histidine | 1.82 | 9.17 | 6.00 | 7.59 |

The above basic amino acids can also be formulated as a functional agent in Part 1 when the pH of the Part 1 solution is not highly acidic and the basic amino acids remains soluble and compatible in Part 1.

For the positively charged peptides in Part 2, any market available, acidic water-soluble peptide that is positively charged at neutral pH and non-toxic can be used. The preferred dosage range for the positively charged peptides is 6-12% w/w.

In Part 1, pH acidifying agents such as phosphoric acid or other acid that is compatible with all ingredients in Part 1 can be used. In Part 2, alkalizing agents such as sodium hydroxide, potassium hydroxide can be used, as long as they are compatible with all ingredients in Part 2.

The flavouring agents that can be used in Part 1 and in Part 2 are substances that trigger the sense of taste and/or smell. They are used to improve the consumer perception and experience in treatment of dentin hypersensitivity. Generally, minty flavours like peppermint oil and spearmint oil are used to give a sense of cleanliness and freshness for dental products. Sweeteners like xylitol or artificial sweeteners like sodium saccharin and sucralose can also be used. A commonly preferred flavouring agent is natural peppermint oil. As long as a flavouring ingredient is compatible with other ingredients in Part 1 and/or Part 2, it can be used at minimum dosage level if desired by applications and users.

Potassium salts can be added to Part 1 and/or Part 2 as quick temporary dentin hypersensitivity desensitizers. Potassium based salts depolarize the excited nerve fibers and numb the pain. Examples of suitable potassium salts include potassium nitrate, potassium citrate, and potassium chloride.

Other functional or inert chemicals may be added to Part 1 and Part 2. Depending on what other functions are desired for the product, functional agents like solvents, such as propylene glycol, and non-ionic and amphoteric surfactants, fluoride, and the like, can be formulated in Part 1 and/or in Part 2 to provide some desired functions for the composition and desensitizing application.

Table 3 below shows an exemplary composition formula for the instantly formed, cationic amino acids based ACP gel. Other formulas can be readily developed following the disclosed formulation approach in this disclosure.

**TABLE 3**

| Part A | |
|---|---|
| Ingredient | % w/w in Part A |
| DI Water | 88.426 |
| Calcium Nitrate | 0.750 |
| Phosphoric Acid, 75% | 0.002 |
| ACULYN^{™} 28 | 10.000 |
| Sodium Lauryl Sulfate (STEPANOL^{®} WA-100 NF/USP ) | 0.422 |
| Natural Peppermint Oil | 0.400 |

| Part B | |
|---|---|
| Ingredient | % w/w in Part B |
| DI Water | 76.479 |
| Sodium Hydroxide | 0.328 |
| Arginine | 8.000 |
| Disodium Phosphate | 0.750 |
| Potassium Nitrate | 10.000 |
| Glycerine | 4.000 |
| Sodium Saccharin USP/FCC | 0.001 |
| Sodium Fluoride USP 0.442 | |

The mix ratio of Part A and Part B in Table 3 is 1:1 (v/v) to create an instantly on-site formed, cationic arginine, ACP gel having a pH range of about 6.5 to about 9.5 for dental treatment of hypersensitive teeth. In general, the gel is stable within a temperature range of about 0°C to about 40 °C for at least 2 hours.

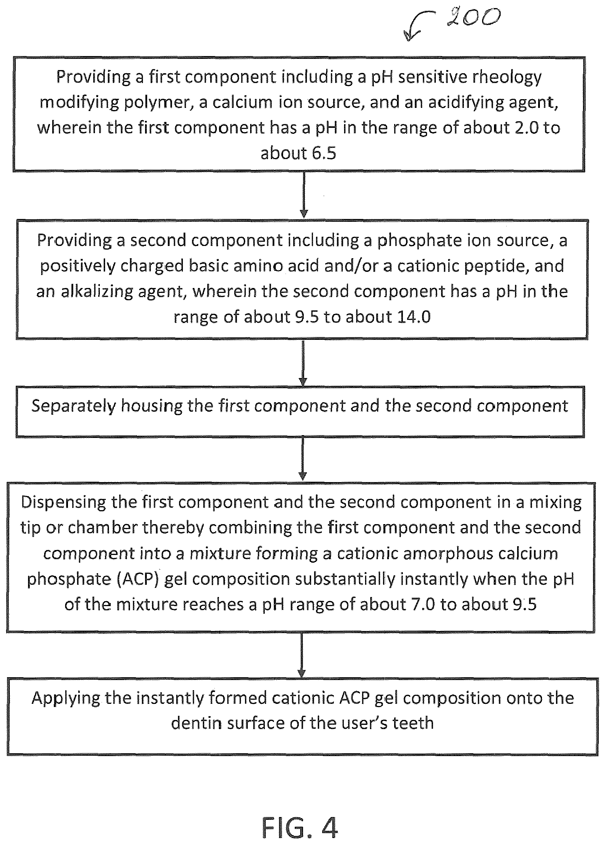

The inventive subject matter further provides an oral health care method for treating dentin hypersensitivity. FIG. 4 is a high-level flow chart of a method 200 for treating dentin hypersensitivity. Method 200 includes providing a first component and a second component. The first component includes a pH sensitive rheology modifying polymer, a calcium ion source, and an acidifying agent. The first component has a pH in the range of about 2.0 to about 6.5. The second component includes a phosphate ion source, a positively charged basic amino acid and/or a cationic peptide, and an alkalizing agent. The second component has a pH in the range of about 9.5 to about 14.0. The method further includes separately housing the first component and the second component, dispensing the first component and the second component in a mixing tip or chamber thereby combining the first component and the second component into a mixture forming a cationic ACP gel composition instantly when the pH of the mixture reaches a pH range of about 7.0 to about 9.5, and applying the instantly formed cationic ACP gel composition onto the dentin surface of the user's teeth.

In some embodiments, the composition can be used as a treatment gel product at the dental office. In other embodiments, the composition can also be manufactured as a sensitivity relief toothpaste for a "take-home" or retail type product for dentin hypersensitivity treatment. In further embodiments, the composition may be used to treat tooth cavities due to the same mechanism and strong affinity to tooth enamel. In addition, the disclosure can be used to produce artificial dentin tissue or artificial tooth material.

Compared to conventional methods and products, the composition according to the inventive subject matter can quickly bring in large quantities of water-soluble calcium and phosphate ions to tooth dentin from non-saliva sources, and speed up the formation of calcium phosphate compounds, which is considered to be the backbone of the occlusion inside the dentin tubules. In a short amount of time, the disclosed composition generates a strong occlusion of dentin tubules by the strong binding complex among dentin, positively charged amino acids/peptide agents, and calcium and phosphate ions or formed ACP particles. The reactions will deposit a dentin-like mineral, as a strong plug within the dentin tubules and form a protective layer on the dentin surface. Since chemically calcium phosphate is more resistant to acid challenge than calcium carbonate in occlusion of dentin tubules, the inventive subject matter is more superior in duration of dentin hypersensitivity relief than existing products.

Those skilled in the art will understand that the disclosed examples may be varied, modified, and altered without departing from the scope of the invention described herein. The present invention may take form in various components and arrangements of components, and in various techniques, methods, or procedures and arrangements of steps.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." In addition, singular references do not exclude a plurality. Thus, references to "a", "an", "first", "second" etc. do not preclude a plurality.

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e*., "one or more" of the elements
so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, *i.e*., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e*., to mean including but not limited to.

## Claims

1. A composition for treating dentin hypersensitivity, comprising:
a first component including a pH sensitive rheology modifying polymer, a calcium ion source, and an acidifying agent, and wherein the first component has a pH in the range of about 2.0 to about 6.5;
a second component including a phosphate ion source, a positively charged basic amino acid and/or a cationic peptide, and an alkalizing agent, and wherein the second component has a pH in the range of about 9.5 to about 14.0; and
wherein the first component and the second component are combined into a mixture, wherein a cationic amorphous calcium phosphate gel composition is created instantly when the pH of the mixture reaches a pH range of about 7.0 to about 9.5.

2. The composition of claim 1, wherein the first component and the second component are combined into a mixture via a mixing tip or a mixing chamber.

3. The composition of claim 1, wherein the pH sensitive rheology modifying polymer is selected from a hydrophobically modified alkali-soluble emulsion polymer of polyacrylic acid, an alkali-soluble emulsion polymer of polyacrylic acid, and a polymer with one or more acidic groups selected from the group consisting of carboxylic acid, sulfonic acid, and phosphoric acid, that is capable of thickening a composition including water upon an increase of the pH of the composition from an acidic to an alkaline value within a pH range of about 7.0 to about 12.0.

4. The composition of claim 1, wherein the calcium ion source is a calcium salt of nitric acid, a calcium salt of aspartic acid, a calcium salt of glutamic acid, or a water-soluble calcium salt of other suitable acid.

5. The composition of claim 1, wherein the acidifying agent is phosphoric acid, or other acid that is compatible with ingredients of the first component.

6. The composition of claim 1, wherein the phosphate ion source is disodium phosphate, monosodium phosphate, or other suitable alkaline water-soluble phosphate salt.

7. The composition of claim 1, wherein the positively charged basic amino acid is arginine, lysine, histidine, or combinations thereof.

8. The composition of claim 1, wherein the cationic peptide is a water-soluble peptide that is positively charged at neutral pH and that is non-toxic to a user.

9. The composition of claim 1, wherein the alkalizing agent is sodium hydroxide, potassium hydroxide, or other suitable alkaline compound.

10. The composition of claim 1, wherein viscosity of the instantly formed cationic amorphous calcium phosphate gel is larger than about 5,000 cP.

11. An oral health care method (200) for treating dentin hypersensitivity, comprising:
providing a first component including a pH sensitive rheology modifying polymer, a calcium ion source, and an acidifying agent, and wherein the first component has a pH in the range of about 2.0 to about 6.5;
providing a second component including a phosphate ion source, a positively charged basic amino acid and/or a cationic peptide, and an alkalizing agent, and wherein the second component has a pH in the range of about 9.5 to about 14.0;
separately housing the first component and the second component;
dispensing the first component and the second component in a mixing tip or chamber thereby combining the first component and the second component into a mixture forming a cationic amorphous calcium phosphate gel composition instantly when the pH of the mixture reaches a pH range of about 7.0 to about 9.5; and
applying the instantly formed cationic amorphous calcium phosphate gel composition onto the dentin surface of the user's teeth.

12. The oral health care method of claim 11, wherein the pH of the first component is in the range of about 2.5 to about 4.5.

13. The oral health care method of claim 11, wherein the pH of the second component is in the range of about 10.5 to about 12.5.

14. A system (10, 100) for preparing a composition for treatment of dentin hypersensitivity, comprising:
a dosing device (12, 112) comprising
a first compartment containing a first component including a pH sensitive rheology modifying polymer, a calcium ion source, and an acidifying agent, and wherein the first component has a pH in the range of about 2.0 to about 6.5, and
a second compartment containing a second component of a phosphate ion source, a positively charged basic amino acid and/or a cationic peptide, and an alkalizing agent, and wherein the second component has a pH in the range of about 9.5 to about 14.0; and
a dispensing device (14, 114) configured to receive the first component and the second component into a mixing tip (18) or chamber (122, 124) and to combine the first component and the second component into a mixture creating a cationic amorphous calcium phosphate gel composition instantly when the pH of the mixture reaches a pH range of about 7.0 to about 9.5, and wherein the dispensing device has an opening to apply the cationic amorphous calcium phosphate gel composition onto a dentin surface of a user's teeth.

15. The system of claim 14, wherein the dosing device includes multiple compartments, each compartment comprising one or more compatible ingredients of the first component and/or one or more compatible ingredients the second component, and wherein the content of the multiple compartments is dispensed into the mixing tip or chamber to form the cationic amorphous calcium phosphate gel composition.
